# EUROPEAN PATENT SPECIFICATION

(11) **EP 3 474 942 B1**
(45) Date of publication and mention of the grant of the patent: **19.07.2023**
(21) Application number: 17816188.1
(22) Date of filing: 22.06.2017
(51) Int. Cl.: A61K 31/568, A61P 19/00, A61P 21/00, A61P 43/00, A61N 1/36

(54) **TESTOSTERONE REPLACEMENT THERAPY IN COMBINATION WITH NEUROMUSCULAR STIMULATION**
TESTOSTERONERSATZTHERAPIE IN KOMBINATION MIT NEUROMUSKULÄRER STIMULATION
THÉRAPIE DE REMPLACEMENT DE TESTOSTÉRONE EN COMBINAISON AVEC UNE STIMULATION NEUROMUSCULAIRE

(30) Priority: 23.06.2016 US 201662353638 P
(43) Date of publication of application: 01.05.2019
(73) Proprietor: Kessler Foundation Inc., East Hanover, NJ 07936 (US); The United States Government as represented by The Department of Veterans Affairs, Washington, DC 20420 (US)
(72) Inventor: FORREST, Gail Florence, Cranbury, NJ 08512 (US); BAUMAN, William A., New Rochelle, NY 10804 (US)
(74) Representative: Hoffmann Eitle
(86) International application number: PCT/US2017/038687
(87) International publication number: WO 2017/223277

(56) References cited:
- WO-A1-2017/123529
- WO-A1-2018/048789
- US-A- 4 569 351
- US-A1- 2010 185 259
- US-A1- 2011 178 572
- US-A1- 2011 224 665
- US-A1- 2012 172 940
- US-A1- 2015 182 784
- US-A1- 2017 197 978
- US-B1- 6 181 965
- US-B2- 8 560 077
- RICHARD K. SHIELDS ET AL: "Musculoskeletal Plasticity After Acute Spinal Cord Injury: Effects of Long-Term Neuromuscular Electrical Stimulation Training", JOURNAL OF NEUROPHYSIOLOGY, vol. 95, no. 4, 1 April 2006 (2006-04-01), pages 2380-2390, XP055658106, US ISSN: 0022-3077, DOI: 10.1152/jn.01181.2005

## Description

### Field of the Invention

The present disclosure generally relates to the field of musculoskeletal rehabilitation following a neurological or neuromuscular injury, such as traumatic or non-traumatic spinal cord injury, stroke or brain injury. More particularly, the invention is defined in the claims and relates to the combination of testosterone replacement therapy and neuromuscular electrical stimulation of multiple muscle groups to treat musculoskeletal atrophy in patients suffering from a neurological disease or disorder.

### Background of the Invention

More than 1.2 million people in the United States have a spinal cord injury ("SCI"), and each year there are 10,000 new cases. It is well appreciated that levels of serum testosterone fall in men after stress, including that which is the result of acute SCI, and levels may be speculated to remain low for variable periods of time, possibly as long as a year or two after the acute event. Risks associated with testosterone deficiency, which include sarcopenia, bone loss, increased cardiovascular disease, as well as unfavorable psychological/mood changes, have been reported. As many as 41% of men between the ages of 18 to 58 with SCI may have low testosterone levels related to immobilization, associated with disuse atrophy of muscle and bone loss.

Muscle atrophy, altered muscle molecular phenotype, and increased intramuscular fat lead to profound adverse musculoskeletal changes and cardiovascular deconditioning following SCI. Deterioration in cardiovascular fitness significantly limits the performance of activities of daily living ("ADL"), may predispose to the development of the metabolic syndrome, and would be anticipated to increase morbidity and mortality. In addition, bone and muscle loss often lead to other direct secondary complications, such as increased fracture risk during transferring, bending, or with minor falls. These "fragility" fractures can often occur without trauma or pain and may not be immediately diagnosed. Furthermore, the atrophy and weakness of the musculoskeletal system may limit the potential for standing or walking. Time post injury and level of injury are good predictors of the severity of osteoporosis and muscle loss.

Understanding the neural and physiological mechanisms of the human spinal cord is essential to the development of effective generalized rehabilitation strategies in patients with neurological impairments. Ultimately, our objective is to provide innovative rehabilitation strategies for patients after SCI that promote greater functional recovery and improved long-term health. While the improvements in both the neuromuscular and skeletal systems are prerequisites to prepare the body for a potential cure, there are major additional health benefits to better metabolic function and improved cardiovascular health.

To date, clinical interventions after SCI have not prevented musculoskeletal deterioration. As such, there is a dire clinical need to improve strategies to preserve muscle of bone.

US 8,560,077 B2, US 2015/182784 A1, US 2011/178572 A1, US 4,569,351 B, US 2011/224665 A1, US 2010/185259 A1 and US 2012/172940 A1 disclose the treatment of musculoskeletal disorders using neuromuscular electrical stimulation.

US 6,181,965 B1 discloses an implantable microstimulator having a coating containing testosterone.

WO 2017/123529 A1, which is prior art pursuant to Article 54(3) EPC, discloses the treatment of symptoms related to a neurodegenerative disorder using neuromuscular electrical stimulation.

J. Neurophysiol., 2006, vol. 95, pp. 2380-2390 discloses the long-term application of neuromuscular electrical stimulation to patients having acute SCI.

### Summary of the Invention

The invention is defined in the claims.

An aspect of the present invention is testosterone for use in a method for treating musculoskeletal deterioration during treatment or recovery of a subject afflicted with a neurological or neuromuscular injury, disease or disorder, the method comprising the steps of:
a) applying the testosterone onto said subject;
b) disposing on said subject surface electrodes for stimulating a plurality of muscle groups; and
c) administering multiple intervention sessions, each of which comprises neuromuscular electrical stimulation to said muscle groups, wherein said neuromuscular electrical stimulation during each intervention session is a wide pulse width, high frequency neuromuscular electrical stimulation (WPHF-NMES), wherein said wide pulse width is 0.5 to 3 milliseconds and said high frequency is 50 to 150 Hz.

The term "method" used herein in the context of the invention should be understood as directed to testosterone for use in the method.

### Detailed Description of the Invention

It is to be understood that the descriptions of the present invention have been simplified to illustrate elements that are relevant for a clear understanding of the present invention, while eliminating, for the purpose of clarity, many other elements found in typical systems and arrangements. Those of ordinary skill in the art will recognize that other elements and/or steps are desirable and/or required in implementing the present invention. However, because such elements and steps are well known in the art, and because they do not facilitate a better understanding of the present invention, a discussion of such elements and steps is not provided herein. The disclosure herein is directed to all such variations and modifications to such elements and methods known to those skilled in the art. Furthermore, the embodiments identified and illustrated herein are for exemplary purposes only, and are not meant to be exclusive or limited in their description of the present invention. The invention is defined in the claims.

As used herein, "TRT" refers to testosterone replacement therapy. "ST" refers to dynamic stand training (defined below). NMES elicits muscle contraction using electrical impulses. In the invention, NMES is used for the neurological rehabilitation in patients with, among other things, SCI. NMES generates contractions by depolarizing axons under stimulating electrodes placed on the skin over a muscle belly or peripheral nerve trunk. Traditionally, NMES is delivered using relatively narrow pulse widths of approximately 0.05 ms to 0.4 ms, and low frequencies of approximately 20 Hz to 40 Hz. This traditional type of NMES favors the activation of motor axons and, thus, generates contractions predominantly through a peripheral pathway that does not involve the central nervous system ("CNS"). Accordingly, traditional NMES recruits motor units in a non-physiological manner, with a random recruitment order of motor unit types and all motor units discharge synchronously.

The inventors believe that musculoskeletal atrophy can be treated in patients suffering from a neurological disease or disorder when testosterone replacement therapy is combined with

NMES of multiple muscle groups and, optionally, a physical activity such as standing. Muscle atrophy below the level of lesion is correlated with the loss of muscle activation following SCI. Muscle loss, especially in larger muscles, may result in energy imbalance (input vs. output) as a result of decreased metabolic rate and increased fat storage. If energy imbalance is not controlled, obesity and other chronic diseases may result. As expected due to increasing loss of muscle mass with higher lesions, level of lesion was correlated with basal metabolic rate. Lack of peripheral sympathetic nervous system response following injury may also contribute to reduced metabolic rate. It has been reported that resting metabolic rate in individuals with SCI is 14-27% lower compared to able-bodied individuals due to decreased muscle mass and depressed sympathetic nervous system activity.

### Muscle Atrophy and Testosterone

Muscle loss is associated with a decline in the secretion of the androgenic hormone testosterone, required for the maintenance of lean body mass and strength. Reductions in testosterone levels are also associated with SCI, suggesting that individuals with SCI are predisposed to age-related changes in body composition. With aging in healthy controls, hypogonadism afflicts approximately 30% of men aged 40-79 years, and its prevalence is associated with aging.

### Muscle Atrophy, Metabolic Factors, Alteration in Fat Mass

Muscle atrophy below the level of lesion is correlated with the loss of muscle activation following SCI. In a prior study, other investigators evaluated the CSA within the first 6 months following injury. They found that muscle CSA was 18-46% lower in SCI than control subjects. The reported losses of muscle CSA were 24% for gastronomies, 12% for soleus, 16% for quadriceps, 14% for hamstrings, and 16% for adductor muscle group. In a prior longitudinal study using dual-energy X-ray absorptiometry ("DXA"), other investigators reported 15% loss of lean mass after first year of injury in the lower limbs, but initial measurements were obtained at about 5 weeks after acute injury¹⁰. The inventors of the present invention have collected preliminary data on lean tissue for over 100 persons with SCI (non-ambulatory and ambulatory) that show a continual decrease in leg lean tissue mass over time, especially for non-ambulatory individuals.

Muscle loss, especially in larger muscles, may result in energy imbalance (input vs. output) as a result of decreased metabolic rate and increased fat storage. If energy imbalance is not controlled, obesity and other chronic diseases may result. As expected due to increasing loss of muscle mass with higher lesions, level of lesion was correlated with basal metabolic rate. Lack of peripheral sympathetic nervous system response following injury may also contribute to reduced metabolic rate. It has been reported that resting metabolic rate in individuals with SCI is 14-27% lower compared to able-bodied individuals due to decreased muscle mass and depressed sympathetic nervous system activity.

Many prior studies have reported significant increases in fat mass following SCI, which is associated with time since injury, age at time of injury, and injury level and completeness. A recent study examined the intramuscular fat CSA and muscle CSA in individuals with motor complete and incomplete SCI at 6 weeks and 3 months post injury and compared these to height and weight of age-matched able-bodied controls. At 6 weeks, the individuals with SCI showed a three-fold increase in intramuscular fat compared to controls, and at 6 months, a 26% increase in intramuscular fat compared to 6 weeks. These findings are relevant especially considering that intramuscular fat is a better predictor of insulin resistance in non-disabled, obese diabetics and non-diabetics compared to that of subcutaneous fat. The higher prevalence of insulin resistance in SCI compared to controls is well documented, and it has been speculated that intramuscular fat explains a predominance of the observed glucose intolerance in those with chronic SCI.

### Bone Adaptations After SCI

Paralysis leads to skeletal unloading and a rapid, linear bone loss within the first several months of injury, especially in the lower limbs. Several studies using DXA or peripheral quantitative computed tomography ("pQCT") have reported that 50% of bone loss will occur within 2 years following SCI. Studies also show that bone mineral density ("BMD") can continue to decrease beyond 7 years post injury, suggesting that there is no plateau to bone loss.

### Bone Structure After SCI

Bone structural changes are an additional consequence of the significant bone loss accompanying SCI. Magnetic Resonance Imaging ("MRI") studies have demonstrated that in those with chronic SCI, reduced bone volume and an increase in trabecular spacing will result. In a cross-sectional study to evaluate the long-term changes in bone metabolism and BMD and fracture incidence after SCI, it was reported that bone loss varies in different compartments. Researchers concluded that at sites with a high proportion of trabecular bone, the bone loss follows a long curve tapering off in 1 to 3 years post injury, but in cortical bone sites such as the tibial diaphysis, bone loss continues even beyond 10 years post injury, and bone is lost at higher rates than other areas. The loss of tibial trabecular bone within 2 years of SCI ranged from 0.4% to 80%, and cortical bone changes ranged from a 1.7% increase to a 33% decrease.

### Bone metabolism after SCI

A comparison between the diverse markers of bone metabolism may prove invaluable in understanding the course of immobilization-related events and the effects of any intervention in the SCI population. As previously reported by other investigators, serum ionized calcium rose above the upper limit of normal and serum parathyroid harmone was suppressed. The markers of bone resorption [total pyridinoline, deoxypyridinoline (free and total), and N-telopeptide] demonstrated a striking rise (up to 10-times the upper limit of normal) after acute immobilization, with highest values between weeks 10 to 16. Patients with quadriplegia had a greater increase in pyridinoline from baseline compared to those with paraplegia. The markers of bone formation (total alkaline phosphatase and osteocalcin) displayed a nonsignificant rise that remained within the normal range.

### Testosterone Effect on Bone

Testosterone is anabolic to bone. The androgen receptor is found in all three bone cells: osteoblasts, osteoclast, and osteocytes. Androgens may promote proliferation and differentiation of osteoblasts, inhibit osteoclast recruitment and bone resorption activity, or affect osteoblast-to-osteoclast signaling, suggesting a role for androgen receptor in regulating bone remodeling. In men with idiopathic osteoporosis, the decrease in testosterone levels correlated with a decline in BMD; TRT increased BMD in the vertebrae. As expected, one recent study in those with acute SCI noted that the bone resorption observed was not found to be related to endocrine abnormalities, probably because of the overwhelming influence of immobilization itself. Nevertheless, the reduced total testosterone and free androgen index levels could be aggravating factors in the development of acute bone loss. In rat models of bone loss due to hind limb unweighting, administration of either testosterone or nandrolone blocked much of the immobilization-related decrease in BMD. Recently, researchers tested whether nandrolone protected bone against loss in male rats that underwent sciatic nerve transaction, followed 28 days later by treatment with nandrolone or vehicle for 28 days. Denervation led to reductions in BMD of 7% and 12% for femur and tibia, respectively

### Risk of Fractures After SCI

Low-energy fractures following commonly performed ADLs, such as transfers or turning in bed, can cause a serious health risk for individuals with SCI. Subsequent complications following bone fractures, including altered healing, pressure sores, infection and oseteomyelitis, increase co-morbidity in these individuals. Also, due to lack of sensation below the injury level and impaired pain sensation, persons with SCI may be unaware of fractures and, therefore, inadvertently delay seeking medical care potentially leading to, complications that prohibit or interfere with fracture treatment. Common fracture sites are around the knee, distal femur and proximal tibia. Fracture rate also increases with the level of injury, as individuals with tetraplegia are at higher risk for fracture or bone injury.

Individuals with SCI not only lose motor and/or sensory function, they also experience dramatic muscle and bone loss. Locomotor training, an activity-based intervention that engages the neuromuscular system below the level of lesion for standing and walking enhances EMG activity and has shown modest improvements in muscle without any attenuation in bone density.

### Combined NMES, TRT and Physical Activity Embodiments of the Invention

TRT has been shown to offer a logical, efficacious, and cost-effective intervention to, in part, counteract these untoward body composition, metabolic, and functional sequelae of relative hypogonadism in those with chronic SCI. In addition, testosterone alone has been shown to increase muscle mass, muscle strength, and bone in androgen-deficient men and older men with low testosterone levels. The inventors believe that the advantageous benefits to those with SCI from the combination of TRT with NMES in accordance with the invention would derive further benefit with the addition of physical activity, such as stand training. In such embodiment of the invention, the model of compressive loads generated during the combination of stand retraining and multi-muscle NMES will be multi-directional and will increase muscle strength and the forces applied to the hip, femur and tibia bones.

Stand training and specifically, dynamic stand training, provides sensory feedback related to standing and bilateral weight bearing, resulting in bilateral muscle activation via central pathways through the spinal cord. Dynamic stand training is accomplished by, for example, participants standing in a dynamic standing frame or standing suspended in a harness by an overhead cable (e.g., body weight support BWS) over a treadmill. Other devices that assisted with balance may be used when needed for dynamic stand training. The dynamic standing training protocol of this embodiment of the present invention is aimed at restoring stable control as in normal standing and involved practicing a series of dynamic movements repetitively while standing. A therapist or other trainers provided manual assistance as needed using specific techniques that will promote the desired motor pattern to facilitate the task. Thus, in accordance with this embodiment of the invention, the stand retraining component provides a physiologically relevant multi-muscle activation through central pathways, dynamic gravity opposed loading of the legs and additional task specific activation of the muscles.

The inventor believes that the model of stand training with TRT will increase muscle mass and strength more than standing alone. The combination of multi-muscle NMES with TRT while stand training will further increase muscle strength compared to either arm alone, resulting in an increased synergistic response of benefit which will surpass the threshold needed to significantly increase bone restoration. This novel, tri-combination synergistic approach of TRT, NMES, and stand training is believed to increase muscle mass and strength to a greater extent than that of each alone or than that of only two of these interventions.

### WPHF-NMES Embodiments of the Invention

The method of the invention facilitates the modulation of the electrophysiological properties of central nervous system ("CNS") circuits during a specific task. These enable the activation of CNS circuits by the combination of intentional supraspinal signals from the participant, afferent signals evoked by the NMES and sensory feedback provided by proprioceptors activated during the task. Together these signals drive plasticity of the nervous system to recover that specific motor function. Also, the consistent activation of CNS circuitry by the method of the invention provides for more torque generation of the muscles and leads to improving the secondary consequences of paralysis, such as cardiovascular and respiratory complications as well as muscle and bone loss.

As described in greater detail in the applicant's U.S. patent application US 2017/0197078 entitled "Neuromuscular Stimulation System and Method", it was discovered that torque generation of a muscle is significantly greater with WPHF-NMES. The inventors of that patent application have documented recovery of arm and hand function in chronic SCI patients treated with WPHF-NMES. Further, the ability to voluntarily move the legs and trunk, as well as improved trunk stability, posture, standing and walking, has been observed in individuals both with upper and motor neuron injury utilizing the method and system described in that patent application.

The inventors of the above referenced patent application unexpectedly discovered additional efficacy when delivering the WPHF-NMES stimulation in such a way as to produce contractions via pathways through the central nervous system using surface electrodes. When contractions are produced utilizing the methods described in that patent application, motor neurons are recruited synaptically, thereby reducing fatigue during voluntary contractions (orderly recruitment, asynchronous discharge). Surprisingly, until methods described in that patent application, few methods have been developed to recruit motor units synaptically, via the electrically-evoked sensory volley, during NMES.

The inventors of the above referenced patent application also discovered that WPHF-NMES incorporating wider pulse durations of, for example, at least approximately 1 ms and higher frequencies of, for example, approximately 100 Hz, which are greater than used traditionally, generates contractions via pathways that traverse the CNS. The WPHF-NMES parameters disclosed herein are, thus, designed to augment the electrically-evoked sensory volley to engage CNS circuits that control movement and produce contractions that are more fatigue-resistant and better for reducing muscle and bone atrophy and improving voluntary control of muscle than contractions generated using traditional NMES.

During an exemplary WPHF-NMES session in accordance with the invention, contractions develop via two pathways. Part of the contraction arises from the well-established peripheral pathway due to the activation of motor axons beneath the stimulating electrodes. However, additional torque develops from the activation of sensory axons which recruits motor units via pathways that travel through the CNS and back to the muscle. This occurs even when the stimulation intensity is adjusted to account for the different pulse durations (thus, overall stimulation charge) and ensure that the stimulation recruits a similar number of motor axons, i.e., similar sized M-waves.

The inventors of the above referenced patent application further discovered that WPHF-NMES generates the additional contraction because a relatively larger sensory volley is sent to the CNS than during traditional NMES. Two reasons account for this. First, sensory axons have a lower rheobase and longer strength duration time constant than motor axons due to differences in the compliment of ion channels on the two types of axons. In this way, when a given number of motor axons are stimulated, i.e., similar sized M-waves, wider pulses recruit a greater proportion of sensory axons, resulting in a greater reflexive contribution to the contraction and more torque. Secondly, the relatively high stimulus frequency of, for example, approximately 100 Hz, sends more impulses to the CNS within a given time period than occurs during traditional NMES.

In addition, the inventors of the above referenced patent application discovered that BMD is increased at the hip and knee when multi-muscle WPHF-NMES sessions are combined with a physical activity such as, for example, dynamic standing. The loading force during dynamic standing with multi-muscle WPHF-NMES can be, for example, approximately 0.7 body weight per limb. The combination of multi muscle WPHF-NMES sessions with a physical activity such as, for example, dynamic standing intervention can also increase muscle strength and muscle volume.

Physiologically, the enhanced synaptic drive generated by WPHF-NMES engages CNS circuits that control movement. In this way, WPHF-NMES recruits motoneurons synaptically and in a more physiologically-relevant manner than traditional NMES. WPHF-NMES recruits motor units according to Henneman's size principle and many motor units discharge asynchronously from one and other. Both of these aspects of motor unit recruitment reduce fatigue of voluntary contractions since fatigue-resistant motor units are recruited first and lower motor unit discharge are required to produce contractions at functionally relevant amplitudes. Further, WPHF-NMES increases the excitability of the pathway that mediates the voluntary command from the brain to muscle. NMES delivered at approximately 100 Hz, but not 50 or 200 Hz, increases the excitability of the corticospinal pathway. This influence of WPHF-NMES on the excitability of CNS circuits that mediate the voluntary command may be responsible in part for the improvements in voluntary control that have been observed using this approach.

### Representative Embodiments of the Invention

The method can further comprise a specific physical activity regime in combination with said neuromuscular electrical stimulation. More than one specific physical activity can be administered. The physical activity can be, without limitation, standing, sitting, sit-to-stand transitioning, walking, weight-bearing, flexing a body part or extending a body part or a combination thereof. Standing can be dynamic standing or standing suspended in a harness.

The testosterone applied to the subject can be in the form of a gel or a patch. The concentration of testosterone in a gel can be 1.62%, which may be dispensed by a pump. In one contemplated embodiment of the invention, after a baseline testing period, TRT will begin in the treatment groups by daily application of 40.5 mg of testosterone gel from a pump. One suitable testosterone gel for this purpose is Androgel^{®} from AbbVie Inc, North Chicago, IL. When one applies transdermal testosterone, it is absorbed and eliminated over the course of a day (hence, the daily administration). To ensure proper dosing, the dose will be titrated based on the pre-dose morning serum testosterone concentration from a single blood draw at baseline, 2 weeks, 1 month and 3 month time-points. As such, follow-up with the participant will be necessary to determine the correct replacement dose of TRT, and thus adjustment of the dose, for example, testosterone gel in the range of approximately 40.5 mg up to 81 mg. If the serum testosterone levels are not within normal physiologic range at the 2-week time point, the dose will be increased in increments of 20.25 mg up to 81 mg.

Alternative testosterone patches and gels useable with the method of the present invention include, for example, Androdem^{®} patches providing a 4 mg/day dose; and Testim^{®}, Axiron^{®}, Fortesta^{®}, Vogelxo^{®} gels.

The neuromuscular electrical stimulation of the invention during each intervention session can have a current amplitude of, for example, no more than 140 mA.

The neuromuscular electrical stimulation during each intervention session is WPHF-NMES. The wide pulse width is from 0.5 to 3 milliseconds and the high frequency is from 50 to 150 Hz.

In certain embodiments of the invention, the wide pulse width can be, for example, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9 or 3.0 milliseconds, or combinations thereof.

In certain embodiments, the high frequency can be, for example, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137,138, 139, 140, 141, 142, 143, 144, 145, 146, 147, 148, 149 or 150 Hz, or combinations thereof.

A WPHF-NMES session produces contractions at least in part via pathways through the central nervous system of the subject. A single session of WPHF-NMES can include, e.g., an initial increase in frequency from a low frequency to the high frequency, then remaining at this high frequency to produce a muscle contraction appropriate for the specific physical activity. In another embodiment, the WPHF-NMES session may further include one or more rest periods. In one embodiment, the low frequency is less than approximately 5 Hz. In another embodiment, the low frequency can be direct current (DC).

In a further embodiment of the invention, the number of intervention sessions are administered until a target level of recovery is achieved. The target level depends on a number of clinical factors such as, for example, the type of injury that resulted in paralysis, the extent of the injury, the age of the subject and other physiological parameters. For example, the neuromuscular injury, disease or disorder results in paralyses can be a traumatic and/or non-traumatic spinal cord injury, stroke or brain injury.

As mentioned above, the WPHF-NMES intervention can be combined with a physical activity regime common in the neuro-rehabilitative arts. In one embodiment, more than one specific physical activity is administered. The physical activity can be, without limitation, standing, sitting, sit-to-stand transitioning, walking, weight-bearing, flexing a body part or extending a body part or a combination thereof. In one embodiment, standing can be dynamic standing or standing suspended in a harness, optionally over a treadmill.

The method of the invention can also include, for example, applying a loading force to the subject during the interventional sessions. An example of a loading force includes a compressive force running parallel to the long axis of the bone.

The inventor unexpectedly discovered that recovery comprises an increase in the force generated by the muscle or a decrease in contraction-fatigue, during voluntary or electrically-evoked contractions in the subject. Thus, in one embodiment of the invention, the method results in a decrease in muscle or bone atrophy in the subject and an increase in voluntary control of skeletal muscles during a movement task.

### Examples

The disclosure is further illustrated by the following examples. It is to be understood that the examples are provided to illustrate certain embodiments and that no limitation to the scope of the disclosure is intended thereby.

### Example 1 (reference example)

### Application of the Testosterone Replacement Therapy (TRT) Gel Pumps

After a baseline testing period, TRT will begin in the treatment groups by application of 40.5 mg of testosterone gel from a pump daily (Androgel, AbbVie Inc, North Chicago, IL, USA). When one applies transdermal testosterone, it is absorbed and eliminated over the course of a day (hence, the daily administration). To ensure proper dosing, the dose will be titrated based on the pre-dose morning serum testosterone concentration from a single blood draw at baseline, 2 weeks and 1 month and 3 month timepoints. As such, follow-up with the participant will be necessary to determine the correct replacement dose of TRT, and thus adjustment of the dose (e.g., 40.5 mg up to 81 mg of gel, i.e. 2 pump presses vs. 3 - 4 pump presses. Thus, if the serum T levels are not within normal physiologic range at the 2-week time point, the dose will be increased in increments of 20.25 mg (1 pump press) up to 81 mg (4 pump presses).

Subjects will be instructed to prime the pump by slowly pushing the pump all the way down three times before the first use of the pump. Any gel released during the priming process should be washed down the sink drain completely to avoid accidental exposure to others. After priming the pump will be ready for use.

To apply the gel, the subject should make sure that their upper arms and shoulders are clean and dry and that there is no broken skin. They should press down firmly on the pump to dispense the gel into the palm of their hand or directly onto the application site (upper arms and shoulders). The subject is to rub the gel onto the upper arms and shoulders which is the area that will normally be covered by a shirt. After applying the gel the subject should immediately wash their hands with soap and water.

The subject is to wait until the gel has completely dried on the skin before putting on a shirt to cover the application area. Application of the gel should be done at the same time every morning. To prevent skin irritation from the T gel, subjects will be instructed on the use of a topical corticosteroid (e.g., triamcinolone) cream at the application site if skin irritation developed.

Table 1 summarizes the daily dose, number of pump presses and application method for testosterone gel:

**Table 1**

| **DOSING INFORMATION FOR ANDROGEL 1.62% PUMP** | | |
|---|---|---|
| Daily dose of Testosterone | Number of Pump Presses | Application Method (Using the palm of the hand) |
| 20.25 mg | 1 pump press | Apply 1 pump press to 1 upper arm and shoulder |
| 40.5 mg | 2 pump presses | Apply 1 pump press to 1 upper arm and shoulder and then apply 1 pump press to the opposite upper arm and shoulder |
| 60.75 mg | 3 pump presses | Apply 2 pump presses to 1 upper arm and shoulder and then apply 1 pump press to the opposite upper arm and shoulder |
| 81 mg | 4 pump presses | Apply 2 pump presses to 1 upper arm and shoulder and then apply 2 pump presses to the opposite upper arm and shoulder |

### Example 2 (reference example)

### Stand Train Alone: Stand Training (Placebo or Testosterone)

All individuals randomized to stand training (with and without ES or testosterone) will train using with body weight support ("BWS") with manual assistance and will undergo a stand evaluation. During the evaluation the participants will be placed on the treadmill in an upright position and suspended in a harness by an overhead cable. A trainer positioned behind the participant will aid in pelvis and trunk stabilization, by applying anterior forces at the pelvis and/or posterior forces at the shoulders, ensuring that the trunk and pelvis are not flexed or hyper-extended. Trainers will assist in maintaining dynamic knee extension by applying posteriorly directed gentle pressure at the patellar tendon to facilitate extensor activity of the weight bearing limbs. Trainers will also promote slight knee flexion and extension to facilitate dynamic weight bearing to enhance neuromuscular activation. All trainers are careful to provide manual assistance only when needed.

Participants will take a break and rest at any time they feel the need to during the training session. During the evaluation the BWS will be lowered until the trainer's assistance is not sufficient to maintain the appropriate kinematics or assure the patient's safety. The amount of BWS and the level of assistance given from each trainer for each body segment will be recorded. The BWS level at which the participant is able to support good standing posture independently will also be recorded. Medial-lateral and anterior-posterior shifts will also be performed on the treadmill. Standing time while on treadmill and overground will be recorded daily as part of the training sessions.

BWS will be given when a subject cannot maintain his body weight while executing limb locomotion. The level of BWS will be adjusted to maximize loading without the knee bending during stance with moderate assistance by a trainer. Manual assistance will be given when the subject (with BWS) cannot perform standing movements relative to the lower limbs and trunk. If required, assistance will be given to aid in knee extension during stance. Assistance will also be given to maintain the pelvis in a stable position and the trunk in an extended position.

Each subject will be encouraged to leave his arms relaxed at the side of the body. To initiate standing, participants will be encouraged to load evenly between the legs, limit pelvis motion (anteriorly/posteriorly or laterally). The leg trainer will aid in knee extension by pushing on the patellar tendon repeatedly to facilitate knee extension and push on the medial hamstring (behind the knee) to prevent hyperextension. The overall goal is to obtain a stable control during stance (and limb control), which resembles normal standing and to practice the movement repetitively. This will be determined by adjusting and ultimately minimizing BWS and minimizing manual assistance.

A circuit training for BWS will be determined for 60 minutes of training [15 minutes of 100% full loading with assistance, 10 minutes of increased BWS for person to do standing without assistance, 10 minutes full body weight load with assistance, increase BWS to stand without assistance , 15 minutes full load with assistance]. The duration of stand retraining sessions will be up to 1 hour or determined by subject's fatigue. Blood pressure ("BP") will be taken continually. To help maintain BP in participants, arm exercises will be used while standing.

If the BP drops below the recommended limit by the examining physician, or the participant complains of dizziness, light headedness, or any autonomic nervous system agitation (nausea, vomiting), the participant will rest seated (feet elevated). After 5 minutes of rest, the participant will recommence the standing again and blood pressure will be monitored. Participants will have three opportunities to complete the 60 minutes of training session. The duration of training and blood pressure will be recorded for further analysis. If a participant cannot tolerate training for 60 minutes, he or she will finish training and training time will be recorded for future analysis. Before each training session, BP will be evaluated (3 consecutive BP measurements within 5 mmHg at 1-minute intervals). Once BP is stable, the participant will begin to train.

### Example 3 (reference example)

### Standing Training and ES: Standing Training and ES (Placebo or Testosterone)

The RT300 functional electrical stimulator, available from Restorative Therapies Inc., Baltimore, MD. USA, is a 6 channel stimulator expandable to 16 channels utilizing wireless single channel stimulators. Each channel of stimulation is individually adjustable including, for example, amplitude 0 to 140 mA; pulse width 50 to 500 µs; frequency from 10 to 100 Hz. Pulse widths up to 3,000 µs are possible in a research mode. The stimulation is controlled via a Windows CE based controller which features a 7 inch color touch sensitive display. An optional I/O port can be configured to provide additional control trigger and timing functions to support research applications. The software included with the RT300 will allow a clinician to generate arbitrary patterns and sequences of complex stimulation for other applications. For our stimulation protocol we will stimulate all muscles of the lower limb including bilateral rectus femoris, bicep femoris, gastrocnemius, tibilias anterior, and gluteaus maximus.

Electrical stimulation will be applied via bifurcated leads and self-adhesive reusable surface electrodes. The electrodes will be applied over the motor points (both legs) on the following muscles: gluteus maximus (GL), rectus femoris (RF), biceps femoris (BF), gastrocnemei (GC), and anterior tibialis (TA) of both legs. Two electrodes will be used for each muscle. One RT300 portable stimulator will be used to induce the electrical stimulation with 10 sets of electrodes for stimulation. In one stimulation protocol, symmetrical biphasic pulses are applied for 300 µs at 35 Hz delivered across a 1000 ohm load, over a duty cycle of 11 seconds on, 60 seconds off, with overlap during each contraction between the upper and lower leg. Therefore, GC, and TA, muscles will be contracted first for 4 seconds. The BF and RF will be contracted next for 7 seconds, while the other musculature are still being stimulated (11 second total). This will be followed by 60 seconds of rest (no stimulation).

This timing and phasing of contractions were selected to promote the muscle groups to contract and relax alternately in an overlapping fashion. Since maximum FES- inducible contraction is required for the venous pump to be effective, subjects will be acclimated to the FES prior to training to find the maximum tolerable level of stimulation for each muscle and apply that during the training. Stimulation will start at the threshold level for minimal contraction and will advance to the maximum contraction over 2 seconds. EMG information may be collected during this time to measure muscle response.

BP and heart rate will be monitored throughout the session. If the BP drops below the recommended limit by the examining physician or clinician, or the participant complains of dizziness, light headedness, or any autonomic nervous system agitation (nausea, vomiting), the participant will rest. After 5 minutes of rest, the participant will recommence the ES again and BP will continue to be monitored. Participants will have three opportunities to complete the 60 minutes of the training session. The duration of training and BP will be recorded for further analysis. The participants will be required to complete 60 minutes of FES-induced contraction of the muscles. If a participant cannot tolerate training for 60 minutes (pulse per second=3 or 4), she/he will finish training and training time will be recorded for future analysis. Before each training session, BP will be evaluated (3 consecutive BP measurements within 5 mmHg at 1-minute intervals). Once BP is stable, the participant will begin to train. BP is continuously collected throughout training.

Electrical stimulation will start while participant is seated and before he is brought up to full standing. BP and heart rate will be monitored during the standing treatment and at pre- and post-standing session. If the BP drops below the recommended limit by the examining physician, or the participant complains of dizziness, light headedness, or any autonomic nervous system agitation (nausea, vomiting), the standing treatment will be discontinued and the participant will be marched in place or brought to the sitting position. The same procedures as described for the other 2 groups will be followed to acclimatize individuals to the ES and standing.

### Example 4 (reference example)

### Stand Training with TRT and ES to Induce Beneficial Changes in Muscle Mass Compared to Other Combinations of These Interventions

***Test* 1:** Muscle Volume will be measured in the thigh. The percent change in muscle volume for the bilateral thigh from before intervention to after intervention will be compared by fitting a linear mixed effects model to the muscle volume data. The use of a mixed effects model is necessitated by the repeated measures nature of the data - each participant will be measured on the left and right sides at each visit. The random effect for the model will be the intercept term, assumed to be random per patient. The mixed effects model will have change in muscle volume as the dependent variable with treatment group as a four-level fixed factor being the primary independent variable. Side (left or right), study site, prior baclofen use, and time since SCI, parameterized as a two-level factor indicating 0.5-1 yr. post-SCI and greater than 1-2 yr. post SCI, will be included as controlling factors, but side, site, and time since SCI comparisons will not be of primary interest. Linear contrasts will be applied to the model to individually compare the ST + ES + TRT group to the three remaining treatment groups (ST, ST + ES, ST + TRT). We will fix the ST + ES + TRT as the control group in the multiple comparison procedure, obviating the need for correction for multiple comparisons.

The mixed effects model allows for inclusion of additional covariates in the model -age and weight among other factors shall be considered as warranted and sample size permitting. These covariates will be control variables rather than as comparisons of interest. It is anticipated that those subjects in the Stand + TRT + ES group will exhibit greater increases in muscle volume than the Stand + TRT, Stand + ES and Stand alone. As a secondary analysis, a mixed effects model identical to the one above shall be employed, but with treatment parameterized as two two-level factors (use of TRT, use of ES) rather than as a single 4-level factor, allowing for the testing of an interaction effect between TRT and ES. Additional analyses will include more general mixed effects models that will include measurements taken at the 3-month follow-up visit. From these models, we will be able to generate point an interval estimates of not only the impact of treatment in the 4 study groups, but also estimates of any post-training effect. Absolute rather than percent change will also be considered as the primary outcome, as noted in the Statistical Contingencies section below.

*Test 2:* Quadriceps muscle torque, muscle cross sectional area will be assessed. Other secondary measures will include BMD for the hip and proximal tibia and distal femur and markers for bone formation and resorption, 3-D volumetric measurement of cortical and trabecular bone in the lower limbs. MRI's of the lower limbs (from above the origin of the iliospsoas to the insertion of achilles tendon) will provide accurate assessments of physiological cross sectional area (PCSA) and volumes of individual muscles. Muscle physiological quadricep muscle torque will be calculated.

BMD will be measured for the hip, distal femur, and proximal tibia. In addition to BMD, 3-D volumetric measurement for cortical and trabecular bone in the lower limbs will be determined. Measures for biochemical markers of bone turnover (resorption/formation) will be performed. The markers for resorption will be urinary cross-linked N-telopeptide of type I collagen (NTx), such as Osteomark, Ostex International, Inc., Seattle, WA,) and serum osteocalcin will be assayed as a marker of formation, such as Diagnostic Systems Laboratories, Inc., Webster, TX.

These tests indicate comparisons of the ST + ES + TRT groups against the remaining three treatment groups. Thus, the analysis shall be a linear mixed effects model with a 4-level factor for the fixed effect term and a single, patient-specific random effect for the repeated measurements (right and left leg) per group. Comparisons of the treatment groups shall be identically carried out - linear contrasts comparing the ST + ES + TRT group to the remaining three groups. Study site, time since SCI, and prior baclofen use shall be included as control variables, and additional covariates (age, weight, etc.) shall be included in these models as warranted, sample size permitting. Additional analyses shall include reparameterization of the fixed effects as two two-level factors (corresponding to ES and TRT) and inclusion of the measurements taken at the follow-up visit, as with the primary outcome.

### Example 5

### Testosterone Replacement Therapy Combined With WPHF-NMES

Testosterone can be applied to the subject as in the examples above. Surface electrodes are placed over muscles or nerves of the lower and/or upper extremities and trunk (legs, trunk, arms and hands). The stimulation is delivered in a frequency-modulated pattern using pulses with a duration equal to or greater than 1 ms and frequency of at least 100 Hz. WPHF-NMES is designed to produce contractions by activating motor and sensory axons, the latter of which activate spinal and other circuits in the central nervous system that control movement.

The temporal parameters of the stimulation are synchronized with the specific motor task being targeted for recovery. WPHF-NMES is combined with training of specific tasks of daily living, as detailed below, to improve motor function to levels that are representative of pre-injury or normal activity with limited use of compensatory movements.

### Dynamic Stand Retraining Combined with WPHF-NMES

Dynamic standing includes, for example, dynamic weight bearing to increase the activation of the muscles of standing while at the same time enhancing the activation of spinal and other circuits in the central nervous system that control movement. The major muscle groups of both legs and trunks are stimulated simultaneously using surface electrodes placed over muscles or nerves of the lower limbs and trunk. The stimulation is delivered in a frequency-modulated pattern using pulses with a duration equal to or greater than 1 ms and frequency of at least 100 Hz. WPHF-NMES is designed to produce contractions by activating motor and sensory axons, the latter of which activated spinal and other circuits in the central nervous system that control movement. Muscles stimulated included the triceps surae, tibilias anterior, quadriceps femoris, hamstrings, gluteus maximus and erector spinae.

Participants are standing in a dynamic standing frame or standing suspended in a harness by an overhead cable (e.g., body weight support) over a treadmill. Other devices that assisted with balance are used when needed. The dynamic standing protocol is aimed at restoring stable control as in normal standing and involved practicing a series of dynamic movements repetitively while standing. A therapist or other trainers provided manual assistance as needed using specific techniques that will promote the desired motor pattern to facilitate the task.

### Sit to Stand Combined with WPHF-NMES

The objective is to transition from a sit to stand position with kinematics at trunk, pelvis and legs that are as close as possible to pre-injury levels. Major muscles of the legs, pelvis and trunk are stimulated using WPHF-NMES in a temporal sequence to facilitate sit to stand. The stimulation is delivered in a frequency-modulated pattern using pulses with a duration equal to or greater than 1 ms and frequency of at least 100 Hz. Muscles stimulated included the triceps surae, tibilias anterior, quadriceps femoris, hamstrings, gluteus maximus and erector spinae.

### Trunk Extension in Sitting Combined with WPHF-NMES

The major muscle groups of the trunk are stimulated in a sequential temporal pattern for trunk extension while seated. The stimulation is delivered in a frequency-modulated pattern using pulses with a duration equal to or greater than 1 ms and frequency of at least 100 Hz. WPHF-NMES is administered to produce contractions by activating motor and sensory axons, the latter of which activated spinal and other circuits in the central nervous system that control movement. The major muscle groups of trunk are stimulated simultaneously using surface electrodes placed over motor points on the muscles of the trunk. The muscles stimulated included the abdominals, upper and lower erector spinae, and trapezius.

### Overhead Press Combined with WPHF-NMES

Muscles of each arm and trunk are stimulated in the sequential pattern appropriate for an overhead press movement. Surface electrodes are placed over the muscles or nerves of the trunk and arms. The stimulation is delivered in a frequency-modulated pattern using pulses with a duration equal to or greater than 1 ms and frequency of at least 100 Hz. WPHF-NMES is designed to produce contractions by activating motor and sensory axons, the latter of which activated spinal and other circuits in the central nervous system that control movement.

## Claims

1. Testosterone for use in a method for treating musculoskeletal deterioration during treatment or recovery of a subject afflicted with a neurological or neuromuscular injury, disease or disorder, the method comprising the steps of:
a) applying the testosterone onto said subject;
b) disposing on said subject surface electrodes for stimulating a plurality of muscle groups; and
c) administering multiple intervention sessions, each of which comprises neuromuscular electrical stimulation to said muscle groups, wherein said neuromuscular electrical stimulation during each intervention session is a wide pulse width, high frequency neuromuscular electrical stimulation (WPHF-NMES), wherein said wide pulse width is 0.5 to 3 milliseconds and said high frequency is 50 to 150 Hz.

2. Testosterone for use according to claim 1, wherein the method further comprises a specific physical activity regime in combination with said neuromuscular electrical stimulation.

3. Testosterone for use according to claim 2, wherein said physical activity is standing, sitting, sit-to-stand transitioning, walking, weight-bearing, flexing a body part, extending a body part or a combination thereof, and wherein said standing is preferably dynamic standing or standing suspended in a harness.

4. Testosterone for use according to claim 1, wherein said testosterone is in the form of a gel or a patch.

5. Testosterone for use according to claim 1, wherein said testosterone is contained in a gel at a concentration of 1.62%.

6. Testosterone for use according to claim 1, wherein said testosterone is applied at a dosage of 40.5 to 81 mg per day.

7. Testosterone for use according to claim 1, wherein said WPHF-NMES produces contractions at least in part via pathways through the central nervous system of said subject.

8. Testosterone for use according to claim 1, wherein a single session of WPHF-NMES comprises an initial increase in frequency from a low frequency to the high frequency of 50 Hz to 150 Hz, remaining at this high frequency to produce a muscle contraction appropriate for a specific physical activity, wherein the low frequency is less than 5 Hz.

9. Testosterone for use according to claim 8, wherein the low frequency is DC.

10. Testosterone for use according to claim 1, wherein said neuromuscular injury, disease or disorder results in paralysis and is traumatic and/or non-traumatic spinal cord injury, stroke or brain injury.

11. Testosterone for use according to claim 1, wherein the method further comprises the step of applying a loading force to said subject during said intervention sessions.

12. Testosterone for use according to claim 1, wherein said subject is a human.

13. Testosterone for use according to claim 12, wherein said human is a quadriplegic, paraplegic or hemiplegic.

## Patentansprüche

1. Testosteron zur Verwendung in einem Verfahren zur Behandlung einer Verschlechterung des Muskelskeletts während der Behandlung oder Genesung eines Subjekts, die an einer neurologischen oder neuromuskulären Verletzung, Krankheit oder Störung leidet, wobei das Verfahren die folgenden Schritte umfasst:
a) Auftragen des Testosterons auf das Subjekt;
b) Anbringen von Oberflächenelektroden zur Stimulation einer Vielzahl von Muskelgruppen an dem Subjekt; und
c) Verabreichen von mehreren Interventionssitzungen, von denen jede eine neuromuskuläre elektrische Stimulation der Muskelgruppen umfasst, wobei die neuromuskuläre elektrische Stimulation während jeder Interventionssitzung eine neuromuskuläre elektrische Stimulation mit einer breiten Pulsbreite und Hochfrequenz (WPHF-NMES) ist, wobei die breite Pulsbreite 0,5 bis 3 Millisekunden beträgt und die Hochfrequenz 50 bis 150 Hz beträgt.

2. Testosteron zur Verwendung gemäß Anspruch 1, wobei das Verfahren ferner ein spezifisches körperliches Aktivitätsregime in Kombination mit der neuromuskulären elektrischen Stimulation umfasst.

3. Testosteron zur Verwendung gemäß Anspruch 2, wobei die körperliche Aktivität Stehen, Sitzen, Übergang vom Sitzen zum Stehen, Gehen, Gewichtsbelastung, Beugung eines Körperteils, Streckung eines Körperteils oder eine Kombination davon ist, und wobei das Stehen vorzugsweise dynamisches Stehen oder in einem Gurtzeug aufgehängtes Stehen ist.

4. Testosteron zur Verwendung gemäß Anspruch 1, wobei das Testosteron in Form eines Gels oder eines Pflasters ist.

5. Testosteron zur Verwendung gemäß Anspruch 1, wobei das Testosteron in einem Gel in einer Konzentration von 1,62% enthalten ist.

6. Testosteron zur Verwendung gemäß Anspruch 1, wobei das Testosteron in einer Dosierung von 40,5 bis 81 mg pro Tag aufgetragen wird.

7. Testosteron zur Verwendung gemäß Anspruch 1, wobei die WPHF-NMES Kontraktionen zumindest teilweise über Wege durch das Zentralnervensystem des Subjekts erzeugt.

8. Testosteron zur Verwendung gemäß Anspruch 1, wobei eine einzelne Sitzung der WPHF-NMES umfasst einen anfänglichen Anstieg der Frequenz von einer Niederfrequenz auf eine Hochfrequenz von 50 Hz bis 150 Hz, verbleiben auf dieser Hochfrequenz, um eine Muskelkontraktion zu erzeugen, die für eine spezifische körperliche Aktivität angemessen ist, wobei die Niederfrequenz weniger als 5 Hz beträgt.

9. Testosteron zur Verwendung gemäß Anspruch 8, wobei die Niederfrequenz Gleichstrom (DC) ist.

10. Testosteron zur Verwendung gemäß Anspruch 1, wobei die neuromuskuläre Verletzung, Krankheit oder Störung zu einer Lähmung führt und eine traumatische und/oder nicht-traumatische Rückenmarksverletzung, ein Schlaganfall oder eine Hirnverletzung ist.

11. Testosteron zur Verwendung gemäß Anspruch 1, wobei das Verfahren ferner umfasst einen Schritt des Aufbringens einer Belastungskraft auf das Subjekt während den Interventionssitzungen.

12. Testosteron zur Verwendung gemäß Anspruch 1, wobei das Subjekt ein Mensch ist.

13. Testosteron zur Verwendung gemäß Anspruch 12, wobei der Mensch ein Tetraplegiker, Paraplegiker oder Hemiplegiker ist.

## Revendications

1. Testostérone pour une utilisation dans un procédé de traitement d'une détérioration musculosquelettique durant un traitement ou une convalescence d'un sujet souffrant d'une lésion, d'une maladie ou d'un trouble neurologique ou neuromusculaire, le procédé comprenant les étapes de :
a) application de la testostérone sur ledit sujet ;
b) mise en place sur ledit sujet d'électrodes de surface pour stimuler une pluralité de groupes de muscles ; et
c) administration de multiples sessions d'intervention, chacune comprenant une électrostimulation neuromusculaire auxdits groupes de muscles, dans laquelle ladite électrostimulation neuromusculaire durant chaque session d'intervention est une électrostimulation neuromusculaire haute fréquence et grande largeur d'impulsion (WPHF-NMES), dans laquelle ladite grande largeur d'impulsion est de 0,5 à 3 millisecondes et ladite haute fréquence est de 50 à 150 Hz.

2. Testostérone pour une utilisation selon la revendication 1, dans laquelle le procédé comprend en outre un régime d'activité physique spécifique en combinaison avec ladite électrostimulation neuromusculaire.

3. Testostérone pour une utilisation selon la revendication 2, dans laquelle ladite activité physique est la position debout, la position assise, le passage de la position debout à la position assise, la marche, le port de poids, la flexion d'une partie du corps, l'extension d'une partie du corps ou une combinaison de ceux-ci, et dans laquelle ladite position debout est de préférence une position debout dynamique ou une position debout suspendu(e) dans un harnais.

4. Testostérone pour une utilisation selon la revendication 1, dans laquelle ladite testostérone est sous la forme d'un gel ou d'un patch.

5. Testostérone pour une utilisation selon la revendication 1, dans laquelle ladite testostérone est contenue dans un gel en une concentration de 1,62 %.

6. Testostérone pour une utilisation selon la revendication 1, dans laquelle ladite testostérone est appliquée en une dose de 40,5 à 81 mg par jour.

7. Testostérone pour une utilisation selon la revendication 1, dans laquelle ladite WPHF-NMES produit des contractions au moins en partie par l'intermédiaire de voies à travers le système nerveux central dudit sujet.

8. Testostérone pour une utilisation selon la revendication 1, dans laquelle une session unique de WPHF-NMES comprend une augmentation initiale de fréquence à partir d'une fréquence faible jusqu'à la fréquence élevée de 50 Hz à 150 Hz, un maintien à cette fréquence pour produire une contraction musculaire appropriée pour une activité physique spécifique, dans laquelle la fréquence faible est inférieure à 5 Hz.

9. Testostérone pour une utilisation selon la revendication 8, dans laquelle la fréquence faible est un CC.

10. Testostérone pour une utilisation selon la revendication 1, dans laquelle ladite lésion, maladie ou trouble neuromusculaire résulte en une paralysie et est une lésion de la moelle épinière traumatique et/ou non traumatique, un accident vasculaire cérébral ou une lésion cérébrale.

11. Testostérone pour une utilisation selon la revendication 1, dans laquelle le procédé comprend en outre l'étape d'application d'une force de charge audit sujet pendant lesdites sessions d'intervention.

12. Testostérone pour une utilisation selon la revendication 1, dans laquelle ledit sujet est un être humain.

13. Testostérone pour une utilisation selon la revendication 12, dans laquelle ledit humain est tétraplégique, paraplégique ou hémiplégique.
